# DEMANDE DE BREVET EUROPEEN

(11) **EP 1 074 238 A1**
(43) Date de publication de la demande: **07.02.2001**
(21) Numéro de dépôt: 00400717.5
(22) Date de dépôt: 15.03.2000
(51) Int. Cl.: A61K 7/02

(54) **Composition de maquillage ou de soin hypoallergénique contenant un organopolysiloxane réticulé à groupement oxyalkyléné, ses utilisations**

(30) Priorité: 30.03.1999 FR 9903969
(71) Demandeur: L'OREAL, 75008 Paris (FR)
(72) Inventeur: Jager-Lezer, Nathalie, 92340 Bourg-la-Reine (FR)
(74) Mandataire: Lhoste, Catherine

(57) **Abrégé**

L'invention se rapporte à une composition hypoallergénique de maquillage ou de soin des matières kératiniques, contenant une phase aqueuse dispersée dans une phase grasse liquide au moyen de particules d'organopolysiloxane solide élastomérique réticulé comportant au moins un groupement oxyalkyléné et notamment un groupement oxyéthyléné, ladite composition étant en outre exempte de co-tensioactif. L'invention se rapporte aussi à l'utilisation de ces particules d'organo-polysiloxane solide élastomérique dans une composition ou pour la fabrication d'une composition de soin, de traitement ou de maquillage hypoallergénique et fraîche, comportant une phase aqueuse dispersée dans une phase grasse liquide.

Cette composition est plus spécialement un rouge à lèvres ou un fond de teint pour le maquillage aussi bien du visage que du corps humain. Cette composition est douce et fraîche à l'application, s'étale facilement, est non-collante et n'est pas desséchante pour la peau et les lèvres.

## Description

La présente invention se rapporte à une composition de soin et/ou de maquillage de la peau et/ou des lèvres des êtres humains présentant à la fois des propriétés hypoallergéniques et de fraîcheur. Cette composition est en particulier une crème de soin, un eye liner, un fard à joues ou à paupières, un fond de teint, ou encore un produit solaire, une crème déodorante, un produit traitant après-shampooing. Elle se présente sous forme d'émulsion eau-dans-huile, huile-dans-eau-dans-huile ou eau-dans-huile-dans-eau, plus ou moins épaisse.

L'invention se rapporte aussi à l'utilisation de particules d'organopolysiloxane particulier dans une composition hypoallergénique pour la peau ou les lèvres.

Les émulsions eau-dans-huile (E/H) présentent l'inconvénient, par rapport à une émulsion huile-dans-eau (H/E) de ne contenir qu'une faible quantité d'eau, et donc d'apporter que peu de fraîcheur à la peau ou les lèvres sur lesquelles elles sont appliquées. Par ailleurs elles confèrent souvent un aspect gras et luisant à la peau, ce qui ne permet pas ou difficilement leur utilisation en milieu chaud et humide et/ou par les utilisateurs à peaux grasses.

Par ailleurs, pour disperser ou émulsionner la phase aqueuse dans la phase grasse liquide, il est nécessaire d'utiliser une certaine quantité de tensioactifs, souvent irritants et mal supportés par les personnes à peau sensibles, pouvant même provoquer des réactions allergéniques.

Il subsiste donc le besoin d'une composition hypoallergénique, non grasse présentant en même temps des propriétés de fraîcheur.

De façon surprenante, la demanderesse a trouvé qu'il était possible de former des compositions hypoallergéniques comportant une émulsion E/H, exemptes de tensioactif et renfermant une grande quantité d'eau, en utilisant des organopolysiloxanes réticulés particuliers comme seul émulsionnant.

Bien qu'il existe des composés du type organopolysiloxane réticulé se dispersant en milieu aqueux, comme par exemple les composés de type KSG 20 ou KSG 21 vendus par la société Shin Etsu, et dont la structure chimique particulière (cf. brevet US-A-5236986 de Shin Etsu) est responsable de cette dispersion en milieu aqueux (présence de groupements polaires leur conférant des propriétés tensioactives), ces composés, contrairement à ceux de la composition de l'invention, ne sont pas capables d'émulsionner de forte quantité d'eau (à savoir jusqu'à 70 % en poids d'eau). Ils ne permettent donc pas l'apport de fraîcheur comme les organopolysiloxanes de la composition selon l'invention.

La présente invention a justement pour objet une composition de soin ou de maquillage de la peau et des lèvres des êtres humains permettant de remédier aux différents inconvénients mentionnés ci-dessus et permettant en particulier l'obtention d'un maquillage ou soin présentant des propriétés cosmétiques améliorées par rapport à celles des produits de l'art antérieur, notamment des propriétés de fraîcheur, de glissant, de non dessèchement de la peau et des lèvres, sans apport de gras, et présentant en outre des propriétés hypoallergéniques et de non irritation de la peau et des lèvres.

L'invention s'applique non seulement aux produits de maquillage des lèvres et de la peau des êtres humains mais aussi aux produits de soin et/ou de traitement des lèvres et de la peau humaine, y compris le cuir chevelu.

Ainsi, l'invention a pour objet une composition hypoallergénique de maquillage ou de soin des matières kératiniques, contenant une phase aqueuse dispersée dans une phase grasse liquide au moyen de particules d'organopolysiloxane solide élastomérique réticulé comportant au moins un groupement oxyalkyléné, ladite composition étant en outre exempte de co-tensioactif.

Par co-tensioactif, on entend tout composé amphiphile capable émulsionner unephase aqueuse dans une phase grasse liquide, ayant une valeur de HLB (balance hydrophile/lipophile) inférieure à 6. La composition selon l'invention contient avantageusement moins de 0,01 % de cotensioactif.

L'invention se rapporte aussi à l'utilisation cosmétique de particules d'organopolysiloxane solide élastomérique réticulé comportant au moins un groupement oxyalkyléné et plus spécialement oxyéthyléné dans une composition cosmétique de soin ou de maquillage, hypoallergénique ou pour personne à peau et/ou lèvres sensibles et/ou réactives, ladite composition étant fraîche et comportant une phase aqueuse dispersée dans une phase grasse.

Par « élastomérique » on entend un matériau souple, déformable ayant des propriétés viscoélastiques et notamment la consistance d'une éponge ou d'une sphère souple. Son modèle d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes de la composition de l'invention contiennent un ou plusieurs groupements oxyalkylénés et en particulier oxyéthylénés (OE), par exemple de 1 à 40 motifs oxyalkylénés, de préférence de de 1 à 20 et mieux de 12 à 20, pouvant former des chaînes polyoxyalkylènes et notamment polyoxyéthylènes. Ces groupements peuvent être pendant, en bout de chaîne ou destinés à lier deux parties de la structure siliconée. Le nombre de silicium portant ces groupements, voire ces chaînes sont avantageusement au nombre de 1 à 10 et mieux de 1 à 6.

Bien que l'invention concerne plus spécialement les organopolysiloxanes à groupements) oxyéthyléné(s) (à savoir ne comportant que des groupements oxyéthylénés comme groupements oxyalkylénés), elle peut aussi concerner les organopolysiloxanes à groupement(s) oxypropyléné(s), c'est-à-dire ne comportant que des groupements oxypropylénés comme groupements oxyalkylénés. Les organopolysiloxanes peuvent aussi comporter à la fois un ou plusieurs groupement(s) oxyéthyléné(s) 1 à 20 (OE) par exemple et un ou plusieurs groupement(s) oxypropyléné(s) (OP) 0 à 20 par exemple ; ces organopolysiloxanes sont aussi appelés des organopolysiloxanes à groupement(s) alkyléthoxy-propyléné(s). De préférence le nombre de groupements oxyéthylénés est supérieur au nombre de groupements oxypropylénés.

Par ailleurs, la structure siliconée formant le squelette polymérique de l'organopolysiloxane à groupement(s) oxyalkyléné(s) est avantageusement une structure polydiméthylsiloxane (PDMS) dont éventuellement une partie des groupes méthyle est substituée par des groupements alkyle en C₂ à C₃₀ et de préférence en C₈ à C₂₄, et mieux de C₁₀ à C₂₀ ou phényle, soit en bout de chaîne soit pendants.

Par ailleurs, l'organopolysiloxane à groupement(s) oxyalkyléné(s) peut comporter un ou plusieurs squelette(s) siliconé(s) relié(s) entre eux par un ou plusieurs groupements oxyalkylénés et de préférence oxyéthylénés tels que définis précédemment ou par un ou plusieurs groupements alkylénés, le nombre de groupement alkyléné allant de 1 à 20 et de préférence de 1 à 10. De préférence, il comporte au moins deux squelettes polymériques liés entre eux.
Avantageusement, le ou les squelettes siliconés de ces organopolysiloxanes de la composition selon l'invention comportent de 26 à 80 atomes de silicium.

Les organopolysiloxanes élastomériques de la composition de l'invention présentent un remarquable pouvoir épaississant de phase grasse liquide et d'émulsification d'une phase aqueuse dans une phase grasse liquide ; ils gonflent dans la phase grasse liquide. Ils ne sont pas desséchants pour la peau et apportent de bonnes propriétés cosmétiques, notamment de douceur, de fraîcheur, de non gras et de facilité d'application. Ces nouveaux élastomères conduisent à des compositions confortables à l'application, de bon étalement, douces et non collantes au toucher. Ces propriétés cosmétiques sont dues, d'une part à la texture des organopolysiloxanes et, d'autre part à leurs propriétés comparables à celles de microéponges piégeant les milieux huileux et en particulier ceux de la composition et ceux sécrétés par la peau.

La composition de l'invention peut se présenter sous forme de pâte, de solide, de crème plus ou moins fluide. Elle peut être une émulsion eau-dans-huile plus ou moins fluide, éventuellement solide, une émulsion multiple et notamment une émulsion eau-dans-huile-dans-eau ou huile-dans-eau-dans-huile. Cette composition peut avoir l'aspect d'une lotion, d'une crème, d'un produit coulé et même se présenter sous forme d'aérosol.

La quantité d'eau peut avantageusement être supérieure à 70 % du poids total de la composition, en vue d'apporter un maximum de fraîcheur.

La composition selon l'invention est stable, c'est-à-dire qu'elle ne démixe à température ambiante pendant au moins deux mois.

Les organopolysiloxanes élastomériques conformes à l'invention sont partiellement ou totalement réticulés et de structure tridimensionnelle. Inclus dans une phase huileuse, ils se transforment, selon le taux de phase huileuse utilisé, d'un produit d'aspect spongieux lorsqu'ils sont utilisés en présence de faibles teneurs en phase huileuse en un gel homogène, en présence de quantités de phase huileuse plus élevées. La gélification de la phase huileuse par ces élastomères peut être totale ou partielle.

Les élastomères de l'invention se présentent sous forme de poudre ou de gel émulsionné contenant un organopolysiloxane élastomère de structure tridimensionnelle, dispersé dans une phase grasse liquide.

Par phase grasse liquide, appelée encore phase huileuse, on entend tout corps non aqueux ou mélange de corps non aqueux, liquides à température ambiante (25°C) et pression atmosphérique (760 mm de Hg).

Les organopolysiloxanes élastomériques selon l'invention peuvent être choisis parmi les polymères réticulés obtenus par réaction d'addition et de réticulation en milieu non aqueux, en présence d'un catalyseur notamment du type platine, d'au moins :
- (a) un premier organopolysiloxane (i) ayant au moins deux groupes vinyliques en position α-ω de la chaîne siliconée ; et
- (b) un second organopolysiloxane (ii) ayant au moins un atome d'hydrogène lié à un atome de silicium par molécule et au moins groupement oxyalkylène notamment oxyéthyléné.

En particulier, l'organopolysiloxane (I) est choisi parmi les polydiméthylsiloxanes (PDMS) et est plus spécifiquement un α-ω-diméthylvinyl polydiméthylsiloxane. L'organopolysiloxane (ii) est choisi notamment parmi les polydiméthylsiloxanes comportant un ou plusieurs atome(s) d'hydrogène, lié chacun à un atome de silicium et un ou plusieurs groupements oxyéthylénés et éventuellement un ou plusieurs groupements oxypropylénés liés, à un atome de silicium via un radical alkylène ayant de 1 à 22 atomes de carbone.

Eventuellement les chaînes silicones des premiers et seconds organopolysiloxanes (i) et (ii) comportent des chaînes pendantes alkyle en C₁ à C₆ et/ou des chaînes aryle.

Les organopolysiloxanes élastomériques de la composition selon l'invention se présente avantageusement sous forme de gel anhydre. Ce gel peut être notamment obtenu comme suit :
- (a) mélange du premier organopolysiloxane (i) et du second organopolysiloxane (ii) ;
- (b) ajout de la phase huileuse au mélange de l'étape (a) ; et
- (c) polymérisation du premier organopolysiloxane (i) et du second organopolysiloxane (ii) en phase huileuse en présence d'un catalyseur de platine.

La phase huileuse utilisée lors de la fabrication du gel anhydre contient une ou plusieurs huiles liquides à températures ambiante (25°C) choisies parmi les huiles hydrocarbonées et/ou les huiles siliconées. Avantageusement, la phase huileuse est une phase liquide siliconée, contenant une ou plusieurs huiles choisies parmi les PDMS à chaîne linéaire ou cyclique liquides à température ambiante comportant éventuellement une chaîne alkyle ou aryle pendante ou en bout de chaîne, la chaîne alkyle ayant de 1 à 6 atomes de carbone.

Par huiles hydrocarbonées, on entend des huiles contenant majoritairement des atomes de carbone et des atomes d'hydrogène et en particulier des chaînes alkyle ou alcényle comme les alcanes, les alcènes. Ces huiles peuvent, en outre, comporter un ou plusieurs groupes ester, éther, hydroxyle ou carboxylique.

Les organopolysiloxanes de l'invention sont en particulier obtenus selon le mode opératoire des exemples 3, 4 et 8 du document US-A-5412004 et des exemples du document US-A-5811487. De préférence, on utilise l'organopolysiloxane de l'exemple 3 du brevet US-A-5412004.

Le produit de l'exemple 3 du document US-A-5412004 se présente sous la forme d'un gel pâteux contenant environ 33 % en poids d'organopolysiloxane réticulé à groupement(s) oxyéthyléné(s) et environ 67 % de PDMS 6cSt. L'organopolysiloxane contient environ 18 % du poids total du polymère d'oxyde d'éthylène.

Le gel élastomérique selon l'invention a un comportement rhéofluidifiant plastique de viscosité sous faible cisaillement voisin de 10⁻⁴s⁻¹ ou 10⁻³s⁻¹allant de 2.10⁶P à 4.10⁶P et une viscosité dynamique de 15 à 50 P (1,5 à 5 Pa.s.) pour une vitesse de cisaillement de 200s⁻¹ à t 10 min, mesurée avec un rhéomètre à contrainte imposée, RS 75 (Haake) à 25°C en géométrie cône/plan ; caractéristique du cône : 20mm de diamètre, 1° d'angle et 40 µm de gap. L'organopolysiloxane de l'invention a, en outre, un comportement viscoélastique à 1 Hz avec un caractère élastique dominant aux faibles valeurs de la contrainte de cisaillement défini comme suit : 800 Pa < G * ₚₗₐₜₑₐᵤ < 2 500 Pa avec δ ₚₗₐₜₑₐᵤ voisin de 10°, G* ₚₗₐₜₑₐᵤ représentant la consistance et δ ₚₗₐₜₑₐᵤ représentant l'élasticité. Il présente un point éclair d'environ 170°C à pression atmosphérique. Pour le gel élastomérique de l'exemple 3 du brevet US-A-5412004, la viscosité dynamique est de 45 P(4,5 Pa.s) à la vitesse de cisaillement de 200s⁻¹.

Le gel élastomérique selon l'invention est, en outre, stable à température ambiante (25°C) au moins 4 mois, sans exsudation d'huile.

De façon préférentielle, ce gel d'organopolysiloxane élastomérique est présent dans la composition à un taux de 0,5 à 99% et mieux de 3 à 75%, ce qui correspond à un taux de polymère en matière active de 0,1 à 33% en poids et mieux de 1 à 25%.

En particulier, les particules d'organopolysiloxane élastomérique (en matière active) ont une taille allant de 0,1 à 500 µm et mieux de 3 à 200 µm et encore mieux de 3 à 50 µm. Ces particules peuvent être sphériques, plates ou amorphes avec, de préférence, une forme sphérique.

L'organopolysiloxane élastomérique de l'invention est en particulier un tensioactif de HLB (Balance Hydrophile Lipophile) d'environ 2,5. Il est donc parfaitement adapté à la fabrication d'émulsion eau-dans-huile stable et d'émulsion huile-dans-eau-dans-huile ou eau-dans-huile-dans-eau, stable. De préférence, l'émulsion est une émulsion simple eau-dans-huile, pour diminuer encore plus les risques de réactions de la part de la peau et des lèvres.

A ce gel d'organopolysiloxane élastomérique peuvent être associés des corps gras liquides à température ambiante, des cires ou des gommes solides à température ambiante, des corps gras pâteux, d'origine animale, végétale, minérale ou synthétique et leurs mélanges.

La phase grasse additionnelle peut être quelconque et contenir des produits liquides à température ambiante comme les huiles siliconées, fluorées, fluorosiliconées, hydrocarbonées éventuellement partiellement siliconées. Ces huiles peuvent être volatiles à température ambiante et pression atmosphérique. Par huile volatile, on entend en particulier une huile susceptible de s'évaporer, en moins d'une heure, au contact de la peau ou des lèvres ayant notamment une pression vapeur non nulle, en particulier allant de 10⁻³ à 300 mm de Hg (à température ambiante et pression atmosphérique) et de préférence supérieure à 0,3 mm de Hg.

Ces huiles peuvent représenter de 1 à 80 % du poids total de la composition, de préférence de 1 à 50 % et mieux de 1 à 30 %.

Comme huiles utilisables dans la composition de l'invention, on peut citer notamment :
- les huiles hydrocarbonées d'origine animale telles que le perhydrosqualène ;
- les huiles hydrocarbonées végétales telles que les triglycérides liquides d'acides gras, par exemple les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel ;
- les huiles de formule R¹COOR² dans laquelle R¹ représente le reste d'un acide gras supérieur comportant de 7 à 19 atomes de carbone et R² représente une chaîne hydrocarbonée ramifiée contenant de 3 à 20 atomes de carbone comme par exemple l'huile de Purcellin, le myristate d'isopropyle, des octanoates, décanoates ou ricinoléates d'alcools ou de polyalcools
- les hydrocarbures linéaires ou ramifiés, d'origine minérale ou synthétique tels que les huiles de paraffine volatiles ou non et leurs dérivés, la vaseline, les polydécènes, le polyisobutène hydrogéné tel que le parléam ;
- les éthers de synthèse de formule R³COR⁴ dans laquelle R³ est un radical alkyle en C₃ à C₁₉ et R⁴ un radical alkyle en C₃ à C₂₀.
- des alcools gras comme l'octyl dodécanol ou l'alcool oléique ;
- les huiles fluorées partiellement hydrocarbonées et/ou siliconées comme les perfluoropolyesters ;
- les huiles siliconées comme les polyméthylsiloxanes à chaîne siliconée linéaire ou cyclique, liquides ou pâteux à température ambiante, les phényl diméthicones, les phényl triméthicones et les polyméthylphénylsiloxanes, les alkylpolydiméthylsiloxanes avec une chaîne alkyle en C₂ à C₂₀.
- leurs mélanges.

Le gel d'organopolysiloxane à groupement(s) oxyéthyéné(s) permet de structurer ces huiles sous forme d'une texture originale type « flan », exempte de gélifiant huileux qui entraverait le toucher doux/soyeux et agréable de la composition.

Avantageusement, la composition selon l'invention peut contenir au moins une cire choisie parmi des cires hydrocarbonées, fluorées, ou siliconées et leurs mélanges, qui peuvent être solides ou semi-solides (sous forme d'une pâte) à température ambiante. Ces cires peuvent être d'origine végétale, minérale, animale et/ou synthétique. En particulier, ces cires présentent une température de fusion commençante supérieure à 25 °C et mieux supérieure à 45 °C, à pression atmosphérique.

Selon l'invention, une cire est un corps gras lipophile, solide à température ambiante, à changements d'état solide/liquide réversible, ayant une température de fusion commençante pourvant aller jusqu'à 200°C et présentant à l'état solide une organisation cristalline anisotrope. En portant la cire à sa température de fusion, il est possible de la rendre miscible à la phase grasse liquide et de former un mélange homogène microscopiquement, puis en ramenant la température du mélange à la température ambiante, on obtient une cristallisation de la cire dans la phase grasse liquide du mélange.

Les cires siliconées peuvent être des cires comportant une structure siliconée et des motifs à une ou plusieurs chaînes alkyle ou alcoxy pendantes et/ou en bout de structure siliconée, ces chaînes étant linéaires ou ramifiées et comportant de 10 à 45 atomes de carbone. Ces cires sont appelées respectivement des alkyldiméthicones et des alcoxydiméthicones. Par ailleurs, ces chaînes alkyle peuvent comporter une ou plusieurs fonctions ester.

Comme autre cires utilisables dans l'invention, on peut citer les cires d'origine animale comme la lanoline, la cire d'abeilles ; les cires d'origine végétale telles que la cire de Carnauba ou de Candellila ; les cires d'origine minérale, par exemple de paraffine, de lignite ou les cires microcristallines, la cérésine ou l'ozokérite ; les cires synthétiques comme les cires de polyéthylène.

Ces corps gras peuvent être choisis de manière variée par l'homme du métier afin de préparer une composition ayant les propriétés, par exemple de consistance ou de texture, souhaitées.

En particulier, la présence de cires permet d'assurer une bonne résistance mécanique, notamment lorsque la composition se présente sous la forme d'un stick.

D'une manière générale, la composition peut comprendre de 0 à 50% du poids total de la composition de cire et de préférence de 5 à 30%.

La composition de l'invention contient une phase aqueuse liquide comportant en particulier de l'eau et des solvants miscibles à l'eau en toute proportion comme les polyols (glycérol, diglycérine, éthylène glycol), les monoalcools inférieurs en C₂ à C₅, l'acétone, et la dicétone. La phase aqueuse peut représenter de 5 à 75 % du poids total de la composition et mieux de 5 à 60 %.

La composition de l'invention peut comprendre, en outre, tout additif usuellement utilisé dans le domaine concerné, tel que des colorants hydrosolubles ou liposolubles, des antioxydants, des huiles essentielles, des conservateurs, des actifs cosmétiques ou dermatologiques, des polymères liposolubles notamment hydrocarbonés tels que les polyalkylènes, des gélifiants de phase aqueuse, des gélifiants de phase grasse, les parfums, les électrolytes comme les sels divalents ou monovalents (NaCI, MgCl₂, MgSO₄).

Ces additifs peuvent être présents dans la composition selon les quantités habituellement utilisées et par exemple à raison de 0 à 20% du poids total de la composition et mieux de 0,1 à 10%. Pour les électrolytes, on utilise en particulier au moins 30 à 60 milliosmoles.

Avantageusement, la composition de l'invention contient comme additifs un ou plusieurs gélifiants de phase aqueuse, à savoir des composés capables de donner l'aspect d'un gel à la composition et de l'épaissir. Parmi les gélifiants de phase aqueuse utilisables selon l'invention, on peut citer : les gélifiants cellulosiques hydrosolubles tels que l'hydroxyéthylcellulose, la méthylcellulose, l'hydroxypropylcellulose, la carboxyéthylcellulose et la carboxyméthylcellulose ; la gomme de guar ; la gomme de guar quaternisée ; les gommes de guar non-ioniques comprenant des groupements hydroxyalkyle en C₁-C₆ ; les gommes de xanthane, de caroube, de scléroglucane, de gellane, de rhamsan, de karoya ; les alginates, la maltodextrine, l'amidon et ses dérivés, l'acide hyaluronique et ses sels ; les argiles et notamment les montmorillonites, les hectorites ou bentones, les laponites ; les polymères à groupement carboxylique comme les acides polyacryliques réticulés au moins partiellement neutralisé tels que les «Carbopol » ou «Carbomer» de la Société Goodrich (Carbomer 980 par exemple neutralisé par de la triéthanolamine -TEA en abréviation-) ; les polymères poly(méth)acrylates de glycéryle ; la polyvinylpyrrolidone ; l'alcool polyvinylique ; les polymères et les copolymères réticulés d'acrylamide ; les homopolymères réticulés de chlorure de méthacryloyloxyéthyltriméthylammonium ; les polyuréthanes associatifs.

Selon l'invention, le gélifiant de phase aqueuse est de préférence choisi parmi la gomme de xanthane, les argiles (bentone ou laponite), les polyuréthanes associatifs, les épaississants cellulosiques, notamment l'hydroxyéthyl cellulose, et les acides polyacryliques réticulés au moins partiellement neutralisés.

Bien entendu l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée. En particulier, ces additifs ne devront pas nuire à l'homogénéité, la stabilité, le confort, la fraîcheur, et l'hypoalergénicité de la composition de l'invention.

La composition selon l'invention peut se présenter sous la forme d'un produit coloré et notamment de maquillage de la peau, en particulier un fond de teint, un fard à joues ou à paupières, un eye-liner, un stick anti-cernes, un produit de maquillage du corps (tatouage), un maquillage des phanères comme un mascara, un vernis à ongles ou d'un produit de maquillage des lèvres comme un rouge à lèvres. Elles peuvent également se présenter sous forme non colorée, contenant éventuellement des actifs cosmétiques ou dermatologiques. Elle peut alors être utilisée comme base de soin pour les lèvres (baumes à lèvres, protégeant les lèvres du froid et/ou du soleil et/ou du vent) ou base fixante à appliquer sur un rouge à lèvres classique, (la base fixante formant alors un film protecteur sur le film de rouge, qui en limite le transfert).

La composition de l'invention peut également se présenter sous la forme d'une composition dermatologique ou cosmétique de traitement ou de soin de la peau (y compris le cuir chevelu), de fibres kératiniques (cheveux, cils, sourcils), des ongles ou des lèvres, ou sous forme d'une composition de protection solaire ou de bronzage artificiel, ou encore sous forme d'un produit nettoyant ou démaquillant de la peau ou des fibres kératiniques, un produit déodorant.

Bien entendu la composition de l'invention doit être cosmétiquement ou dermatologiquement acceptable, à savoir non toxique et susceptible d'être appliquée sur la peau (y compris l'intérieur des paupières) ou les lèvres d'êtres humains.

De façon préférentielle, la composition de l'invention peut comprendre une phase particulaire, généralement présente à raison de 0 à 60 % du poids total de la composition, de préférence de 5 à 35 %, et qui peut comprendre des pigments et/ou des nacres et/ou des charges habituellement utilisés dans les compositions cosmétiques.

Par pigments, il faut comprendre des particules blanches ou colorées, minérales ou organiques, insolubles dans le milieu de la composition, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent à modifier la texture de la composition ainsi que l'effet de matité/brillance.

Les pigments peuvent être présents dans la composition à raison de 0 à 60 % du poids de la composition finale, et de préférence à raison de 4 à 25 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium. Les pigments peuvent être enrobés ou non.

Les nacres peuvent être présentes dans la composition à raison de 0 à 20 % du poids total de la composition, de préférence à un taux de l'ordre de 2 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 35 % du poids total de la composition, de préférence 0 à 15 %. On peut notamment citer le talc, le mica, la silice, le kaolin, les poudres de Nylon (Orgasol notamment) et de polyéthylène, le Téflon, l'amidon, le nitrure de bore, des microsphères de copolymères telles que l'Expancel (Nobel Industrie), le Polytrap (Dow Corning) et les microbilles de résine de silicone (Tospearl de Toshiba, par exemple) et les fibres par exemple de polyamides.

L'organopolysiloxane à groupement(s) oxyethyléné(s) présente l'avantage de bien disperser les pigments et de conférer ainsi, un maquillage homogène. Il présente en outre, l'avantage de stabiliser de façon remarquable les compositions de l'invention.

La composition selon l'invention peut être fabriquée à froid ou par chauffage d'un ou plusieurs organopolysiloxanes élastomériques sous forme de gel anhydre, ajout d'un ou plusieurs pigments et/ou d'un ou plusieurs autres additifs, ajout éventuel de corps gras fondus (notamment portée à la température de fusion des cires la plus élevée), ajout de la phase aqueuse, puis émulsification.

L'invention a encore pour objet l'utilisation de particules d'organopolysiloxane solide élastomérique réticulé comportant au moins un groupement d'oxyalkylène notamment d'oxyéthylène dans une composition cosmétique ou pour la fabrication d'une composition de traitement, de maquillage ou de soin de la peau ou des lèvres, hypoallergénique et fraîche, comportant une phase aqueuse dispersée dans une phase grasse liquide, la composition étant destinée aux personnes à peau et/ou lèvres sensibles et/ou réactives.

L'invention a encore pour objet un procédé cosmétique pour augmenter l'apport de fraîcheur et d'hypoallergénécité d'une composition cosmétique comportant une phase aqueuse dispersée dans une phase grasse liquide, consistant à émulsionner la phase aqueuse dans la phase grasse liquide à l'aide de particules d'un organopolysiloxane élastomérique réticulé telles que définies précédemment.

L'invention est illustrée plus en détail dans les exemples suivants. Les pourcentages sont donnés en poids.

### Exemple 1 : Réalisation d'un font de teint hypoallergénique à texture « flan »

- Phase grasse
   ◆ Huile de silicone (PDMS, 6 cSt) 10%
   ◆ Pigment 10%
   ◆ Silicone modifiée (Exemple 3 du brevet US-A-5412004) 18% (5,7 en matière active)
- Phase acqueuse
   ◆ Conservateur qs
   ◆ Sel divalent 0,4%
   ◆ Eau qsp 100%

### Résultat :

Obtention d'un fond de teint de texture gel type « flan» (dessert), d'une grande fraîcheur à l'application, de bonne tenue dans le temps, exempte de tensioactif autre que l'élastomère, et de co-émulsionnant.

### Préparation :

On fait gonfler l'organopolysiloxane dans l'huile à température ambiante ensuite on ajoute les pigments, la phase aqueuse, puis on mélange le tout sous agitation, jusqu'à émulsification de la phase huileuse dans la phase aqueuse.

### Exemple 2 : Réalisation d'une crème de soin hypoallergénique

- Phase grasse
   ◆ Silicone modifiée (Exemple 3 du brevet US-A-5412004) 18%
   ◆ Huile de silicone (PDMS, 6cSt) 10%
- Phase aqueuse
   ◆ Conservateur qs
   ◆ Sel divalent 0,7%
   ◆ Eau qsp 100%

### Résultat :

Obtention d'une crème non grasse de consistance type « entremet », très fraîche, de bonne tenue dans le temps. Cette crème contient plus de 70 % d'eau, sans ajout de tensioactif autre que l'élastomère, ni de co-émulsionnant.

### Préparation :

On prépare cette composition comme dans l'exemple 1.

## Revendications

1. Composition hypoallergénique de maquillage ou de soin des matières kératiniques, caractérisée en ce qu'elle contient une phase aqueuse dispersée dans une phase grasse liquide au moyen de particules d'organopolysiloxane élastomérique solide réticulé comportant au moins un groupement oxyalkylène, ladite composition étant en outre exempte de co-tensioactif.

2. Composition selon la revendication 1, caractérisée en ce que l'organopolysiloxane élastomérique comporte au moins un groupement d'oxyéthylène.

3. Composition selon la revendication 1 ou 2, caractérisée en ce que l'organopolysiloxane élastomérique ne comporte que des groupements oxyéthylénés comme groupements oxyalkylénés.

4. Composition selon l'une des revendications précédentes, caractérisée en ce que l'organopolysiloxane élastomérique est obtenu par réaction d'addition et de réticulation en milieu non aqueux, en présence d'un catalyseur notamment de type platine, d'au moins : - un premier organopolysiloxane (i) ayant au moins deux groupements vinyliques en position α-ω de la chaîne siliconée par molécule ; et - un second organopolysiloxane (ii) ayant au moins un atome d'hydrogène lié à un atome de silicium par molécule et au moins un groupement oxyalkyléné et notamment oxyéthylèné.

5. Composition selon l'une des revendications précédentes, caractérisée en ce que le premier organopolysiloxane (i) est choisi parmi les polydiméthylsiloxanes.

6. Composition selon l'une des revendications 4 à 5, caractérisée en ce que le premier organopolysiloxane (i) est un α-ω-diméthylvinyl polydiméthylsiloxane.

7. Composition selon l'une des revendications 4 à 6, caractérisée en ce que le second organopolysiloxane (ii) est choisi parmi les polydiméthylsiloxanes comportant un ou plusieurs atomes d'hydrogène et un ou plusieurs groupements oxyalkylénés notamment oxyéthylènés, liés à un atome de silicium via un radical alkylène ayant de 1 à 22 atomes de carbone.

8. Composition selon l'une quelconque des revendications précédentes, caractérisée en ce que les particules d'organopolysiloxane sont sous forme d'un gel anhydre obtenu selon les étapes suivantes :
- (a) mélange du premier et second organopolysiloxanes (i) et (ii) ;
- (b) ajout de la phase grasse liquide au mélange de l'étape (a) ;
- (c) polymérisation du premier et second organopolysiloxanes (i) et (ii) en phase grasse liquide en présence d'un catalyseur de platine.

9. Composition selon l'une des revendications précédentes, caractérisée en ce que les particules d'organopolysiloxanes sont sous forme d'un gel à comportement viscoélastique à 1 Hz de valeur de contrainte de cisaillement telle que 800 Pa < G * ₚₗₐₜₑₐᵤ < 2 500 Pa avec un δ ₚₗₐₜₑₐᵤ de valeur de 10°, G * ₚₗₐₜₑₐᵤ représentant la consistance et δ ₚₗₐₜₑₐᵤ l'élasticité.

10. Composition selon l'une des revendications précédentes, caractérisée en ce que les particules d'organopolysiloxanes ont une taille allant de 3 à 200 µm et mieux de 3 à 50 µm.

11. Composition selon l'une quelconque des revendications précédentes, caractérisée en ce que la phase grasse liquide contient une ou plusieurs huiles liquides hydrocarbonées et/ou siliconées.

12. Composition selon l'une des revendications précédentes, caractérisée en ce que la composition contient, entre autre, au moins un corps gras choisi parmi les huiles volatiles ou non, les cires, les gommes et les corps gras pâteux, d'origine animale, végétale, minérale ou synthétique, et leurs mélanges.

13. Composition selon l'une des revendications précédentes, caractérisée en ce que l'organopolysiloxane élastomérique représente de 0,1 à 33 % du poids total de la composition de préférence de 1 à 25 %.

14. Composition selon l'une des revendications précédentes, caractérisée en ce que la phase aqueuse représente de 5 à 75 % et mieux de 5 à 60 % du poids total de la composition.

15. Composition selon l'une des revendications précédentes, caractérisée en ce que la phase grasse liquide représente de 1 à 80 % du poids total de la composition, de préférence de 1 à 50 %.

16. Composition selon l'une des revendications précédentes, caractérisée en ce qu'elle comprend, en outre, une phase particulaire présente à raison de 0 à 60 % du poids total de la composition, de préférence 5 à 35 %.

17. Composition selon l'une des revendications précédentes, caractérisée en ce qu'elle contient, en outre, au moins un actif cosmétique ou dermatologique.

18. Composition selon l'une des revendications précédentes, caractérisée en ce qu'elle est sous forme d'émulsion simple.

19. Composition selon l'une des revendications précédentes, caractérisée en ce qu'elle se présente sous forme d'une composition de fond de teint, de fard à joues ou à paupières, d'un produit anti-cernes, d'un rouge à lèvres, d'un eye-liner, d'un mascara, d'un produit de maquillage du corps, d'une base de soin ou d'une base fixante pour les lèvres, d'un produit dermatologique ou de soin de la peau ou des fibres kératiniques, d'une composition de protection solaire ou de bronzage artificiel, d'un produit nettoyant de la peau ou des fibres kératiniques, une crème déodorante.

20. Composition selon l'une des revendications précédentes, caractérisée en ce qu'elle contient, en outre, au moins un ingrédient choisi parmi les conservateurs, les parfums, les gélifiants de phase aqueuse, les électrolytes.

21. Utilisation de particules d'organopolysiloxane solide élastomérique réticulé comportant au moins un groupement oxyalkyléné et notamment oxyéthyléné dans une composition cosmétique ou pour la préparation d'une composition de soin, de traitement ou de maquillage, hypoallergénique et fraîche, destinée aux personnes à peau et/ou lèvres sensibles et/ou réactives.

22. Utilisation cosmétique de particules d'organopolysiloxane solide élastomérique réticulé comportant au moins un groupement d'oxyalkylène et notamment d'oxyéthylène dans une composition cosmétique de maquillage, de traitement ou de soin de la peau ou des lèvres, hypoallergénique et fraîche, comportant une phase aqueuse dispersée dans une phase grasse liquide.

23. Utilisation selon la revendication 21 ou 22, caractérisée en ce que les particules d'organopolysiloxane élastomérique sont obtenues selon l'une des revendications 4 à 8.

24. Procédé cosmétique pour augmenter l'apport de fraîcheur et d'hypoallergénésité d'une composition cosmétique comportant une phase aqueuse dispersée dans une phase grasse liquide, consistant à émulsionner la phase aqueuse dans la phase grasse liquide à l'aide de particules d'un organopolysiloxane élastomérique réticulé comportant au moins un groupement oxyalkyléné et notamment oxyéthyléné.
